# EUROPEAN PATENT SPECIFICATION

(11) **EP 2 786 732 B1**
(45) Date of publication and mention of the grant of the patent: **04.01.2017**
(21) Application number: 12852907.0
(22) Date of filing: 06.11.2012
(51) Int. Cl.: A61F 13/20

(54) **MENSTRUAL TAMPON POSITION-CONFIRMING SHEET, EXPLANATORY LEAFLET FOR MENSTRUAL TAMPON, PACKAGING BAG FOR MENSTRUAL TAMPON, AND MENSTRUAL TAMPON-STORING OBJECT**
MENSTRUALTAMPON POSITIONSBESTÄTIGUNGSFOLIE, ERLÄUTERNDER BEIPACKZETTEL FÜR DEN MENSTRUALTAMPON, VERPACKUNGSBEUTEL FÜR DEN MENSTRUALTAMPON UND MENSTRUALTAMPONAUFBEWAHRUNGSOBJEKT
COUCHE DE CONFIRMATION DE POSITION DE TAMPON MENSTRUEL, NOTICE EXPLICATIVE POUR TAMPON MENSTRUEL, SACHET D'EMBALLAGE POUR TAMPON MENSTRUEL, ET OBJET DE STOCKAGE DE TAMPON MENSTRUEL

(30) Priority: 30.11.2011 JP 2011263053
(43) Date of publication of application: 08.10.2014
(73) Proprietor: Unicharm Corporation, Ehime 799-0111 (JP)
(72) Inventor: TANIGUCHI, Kenta, Kanonji-shi Kagawa 769-1602 (JP); ITO, Yukihiro, Kanonji-shi Kagawa 769-1602 (JP); YAMAKI, Kouichi, Kanonji-shi Kagawa 769-1602 (JP); MATSUSHIMA, Azusa, Kanonji-shi Kagawa 769-1602 (JP)
(74) Representative: Peter, Julian
(86) International application number: PCT/JP2012/078707
(87) International publication number: WO 2013/080758

(56) References cited:
- WO-A2-2007/146270
- JP-A- 2003 175 076
- JP-A- 2005 211 428
- US-A1- 2004 054 317
- US-A1- 2005 171 463

## Description

### [Technical Field]

The present invention relates to a position confirmation sheet of a sanitary tampon, etc., which enables a user to check whether or not an inserted absorber of a sanitary tampon is disposed at an appropriate position.

### [Background Art]

The sanitary tampon has an absorber that is disposed inside the vagina to absorb the bodily fluid, and has a strand member extending out from the absorber. In using a sanitary tampon, a user disposes the absorber at an appropriate position in the vagina by using the applicator for a tampon One end of the strand member of the sanitary tampon is disposed inside the vagina together with the absorber while the other end is disposed outside the body.

In a sanitary tampon described in Patent Literature 1 has a strand member having a colored portion (see Fig. 2, for example). The colored portion extends from a center portion of the strand member to a tip of the strand member. A user can visually check the colored portion of the strand member with the absorber being disposed in the vagina

When the absorber is disposed at an appropriate position in the vagina the colored portion is positioned at a user side, whereas the colored portion is positioned farther from the user when the absorber is not disposed at an appropriate position in the vagina (when the insertion depth is shallow) than the position with the absorber being disposed at an appropriate position. In this way, the user can confirm the insertion depth of the absorber by checking the position of the colored portion of the strand member.

### [Prior Art]

### [Patent Literature]

[Patent Literature 1] Japanese Unexamined Patent Application Publication No. 2003-175076

Further prior art in this technical field is disclosed in document WO 2007/146270 A2.

### [Summary of Invention]

However, the applicant has found the following problems in the above-described sanitary tampon.

The strand member is disposed closely to the vaginal opening when the absorber is inserted in the vagina, so that the strand member may become dirty with menstrual blood or the like. When the strand member gets dirty with menstrual blood or the like, it may become difficult to visually detect a marker for checking a state of the inserted absorber.

Further, the strand member is partially disposed inside the body when the absorber is disposed in the vagina. When the strand member to be inserted in the body is colored, some users may concern hygienic problem that might be caused by the use of a coloring agent or the like. In addition, some users may desire to ensure that the colored portion is not inserted into the body, which may cause an insufficient insertion of the absorber. That is, the strand member having the colored portion may rather prevent a user from disposing the absorber at an appropriate position.

Moreover, when the absorber is inserted by using an applicator for a tampon, the colored portion of the strand member is covered with the applicator, so that a user may not be able to visually observe the colored portion. Consequently, the user may fail to check whether or not the absorber is disposed at an appropriate position when the absorber is inserted.

Thus, the present invention has been made in view of the foregoing problems. An object of the present invention is to provide a position confirmation sheet of a sanitary tampon, etc., which enables a user to dispose the absorber at an appropriate position by checking an insertion position of an absorber.

In order to solve the above-described problems, the present invention provides a position confirmation sheet (position confirmation sheet 20) of a sanitary tampon, wherein the sanitary tampon comprises an absorber (absorber 11) and a strand member (strand member 12) extending from a tail end of the absorber (tail end 11b) in an insertion direction into a vagina, and the position confirmation sheet is to be used for checking a position of an end of the strand member disposed outside a body with the absorber being inserted into the body, comprising: a disposition indicator unit (disposition indicator unit 21) that indicates a position for aligning the confirmation sheet along the strand member disposed outside the body with the absorber being inserted into the body; and an insertion position indicator unit (insertion position indicator unit 22) that indicates an appropriate position of the end of the strand member (strand tail end 12d) disposed outside the body when the absorber is appropriately inserted into the body and the position confirmation sheet is aligned along the strand member according to the disposition indicator unit.

An explanatory note (explanatory note 30) of a sanitary tampon according to the present disclosure has the above-described position confirmation sheet and a usage indication unit (usage indication unit 33) that indicates a usage mode of a sanitary tampon.

A package bag (package bag 40) of a sanitary tampon according to the present disclosure has the above-described position confirmation sheet and individually contains a sanitary tampon, wherein the insertion position indicator unit is disposed at an outer surface of the package bag.

A container (container 50) according to the present disclosure has the above-described position confirmation sheet and contains a plurality of sanitary tampons, wherein the insertion position indicator unit is disposed at an outer surface of the container.

### [Brief Description of Drawings]

Fig. 1 is a plan view of a sanitary tampon and a position confirmation sheet according to a first embodiment.
Fig. 2 is a lateral view of an applicator-type tampon according to the first embodiment.
Fig. 3 is a schematic cross-sectional view showing the applicator-type tampon and the position confirmation sheet in use according to the first embodiment.
Fig. 4 is a diagram showing the position confirmation sheet in use according to the first embodiment.
Fig. 5 is a plan view of a position confirmation sheet according to a first modification to a fourth modification.
Fig. 6 is a plan view of a position confirmation sheet according to a fifth modification to an eighth modification.
Fig. 7 is a plan view of an explanatory note of a sanitary tampon according to a second embodiment.
Fig. 8 is a diagram showing a package of a sanitary tampon and a container bag of a sanitary tampon according to a third embodiment.

### [Description of Embodiments]

### (First embodiment)

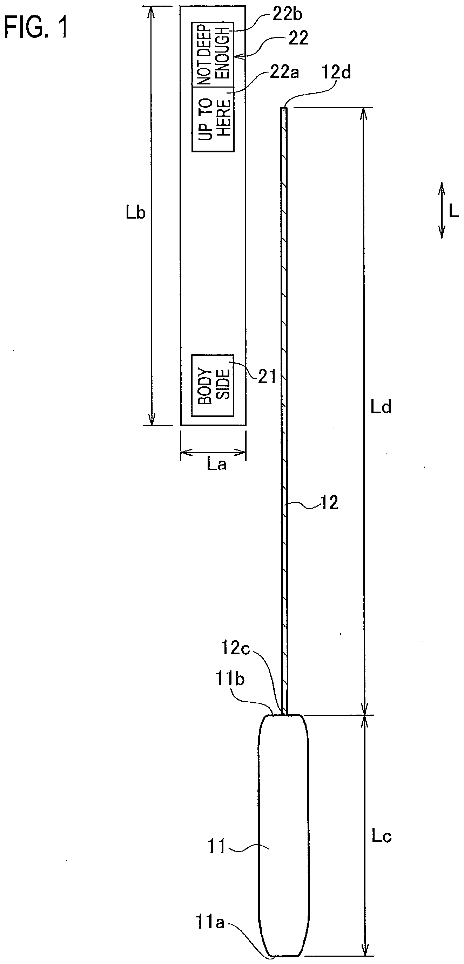

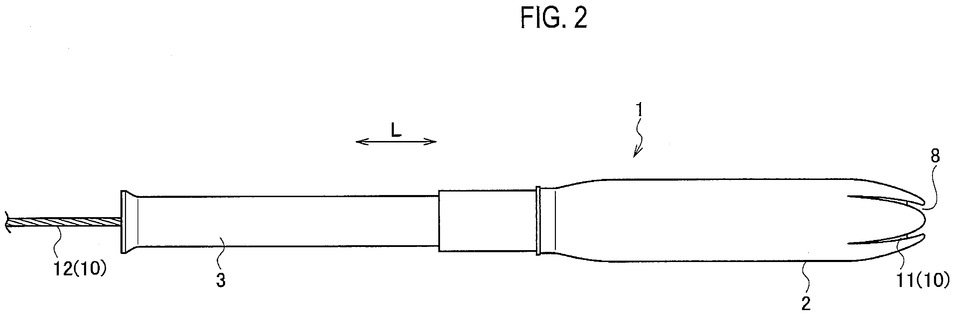

With reference to Fig. 1 to Fig. 4, a position confirmation sheet of a sanitary tampon according to a first embodiment will be explained. Fig. 1 is a plan view of the sanitary tampon and the position confirmation sheet according to the first embodiment. Fig. 2 is a lateral view of an applicator-type tampon.

An applicator-type tampon 1 comprises an applicator for a tampon having an outer tube 2 and an inner tube 3, and a sanitary tampon 10 is contained in the applicator.

The outer tube 2 and the inner tube 3 are cylindrical in shape having a hollow portion therein. The cross-sectional shape of the outer tube 2 and the inner tube 3 is a perfect circle. The outer tube 2 and the inner tube 3 are made of polyolefin such as polyethylene and polypropylene, or a thick paper laminated with a polyolefin film. Although the cross-sectional shape of the outer tube 2 and the inner tube 3 according to the present embodiment is a precise circle, other shape that can be inserted easily inside the vagina, e.g., elliptical, may be employed for the outer tube 2 and the inner tube 3 according to the present invention.

An absorber 11 that configures the sanitary tampon 10 is contained inside the outer tube 2. The sanitary tampon 10 has the absorber 11 and a strand member 12 extending out from the absorber 11. The absorber 11 is positioned at a later-described push-out aperture 8 side of the outer tube 2, and has a tip 11a and a tail end 11b in the direction of insertion into the vagina. The strand member 12 is attached to the absorber 11 by sewing, hooking, or joining with an adhesive, etc.

The strand member 12 runs through the inner tube 3 from the tail end 11b of the absorber 11, extending out from the inner tube 3. A strand tip 12c, which is the end of the strand member 12, is fixed to the absorber 11, and a strand tail end 12d, which is the other end of the strand member 12, is a free end. A user can pull out the inserted absorber 11 from the vagina by pulling the strand member 12.

The absorber 11 can be made from a variety of fluid absorbent materials commonly used for absorbent products such as rayon, cotton, and synthetic fibers. Examples of other materials include creped cellulose wadding, textured synthetic fibers, foams, tissue or laminate thereof and the like, as well as a combination of these materials.

A position confirmation sheet 20 is used for confirming that the absorber 11 is inserted at an appropriate position by observing a position of the end of the strand member 12 disposed outside the body (extending out from the vaginal opening) with the absorber 11 of the sanitary tampon 10 being inserted into the body.

The position confirmation sheet 20 includes a disposition indicator unit 21 that indicates a position for aligning the position confirmation sheet 20 along the strand member 12 disposed outside the body with the absorber 11 being inserted into the body, and an insertion position indicator unit 22 that indicates an appropriate position of the strand tail end 12d when the absorber is inserted into the body and the position confirmation sheet 20 is aligned according to the disposition indicator unit 21.

The position confirmation sheet 20 is made of a sheet of paper in the embodiment, but it may be made from other materials such as cardboard paper, laminate, urethane foam, silicone, rubber, or the like. The position confirmation sheet 20 according to the present embodiment has an ink-jet printed surface.

The disposition indicator unit 21 indicates a position and an orientation of the position confirmation sheet relative to a body of a user when the position confirmation sheet is used. In the present embodiment, a portion that is to be located at a body side (vaginal opening side) of the user is indicated with a text "body side". It is noted that the disposition indicator unit 21 may show other texts than body side such as vaginal side and strand end side as well as an illustration of a strand, a sanitary tampon, etc., as far as the disposition indicator unit 21 is configured to indicate a position or a orientation for disposing it.

The insertion position indicator unit 22 indicates an appropriate position of the strand member. In the present embodiment, there are an appropriate position indicator unit 22a that indicates a text "up to here" at a portion where the strand tail end 12d falls when the absorber 11 is inserted appropriately, and an inappropriate position indicator unit 22b that indicates a text "not deep enough" at a portion where the strand tail end 12d falls when the absorber 11 is not inserted deeply enough.

It is noted that the disposition indicator unit 21 and the insertion position indicator unit 22 may be imparted not only with an information indication unit that indicates a position, etc., but also with color, a pattern, texts, or lines. Further, a planar shape of the position confirmation sheet is not limited to a rectangle, and may be in the form of ellipse, circle, square, diamond, arrow, flower, shooting start, or the like.

A dimension La in a widthwise direction of the position confirmation sheet preferably is at least 5 mm to facilitate a user's handling of the position confirmation sheet and to secure a space for the disposition indicator unit. If the dimension La in a widthwise direction of the position confirmation sheet is too long, a user may have difficulty in handling and holding the sheet at a crotch. Therefore, the dimension in a widthwise direction of the position confirmation sheet desirably is equal to or less than 20 mm.

A dimension Lb in a longitudinal direction of the position confirmation sheet may be such that it covers the length of the strand member extending from the vaginal opening, and may be about 100 mm to 200 mm, for example. In the present embodiment, the dimension La in a widthwise direction of the position confirmation sheet is 7 mm, and the dimension in a longitudinal direction is 150 mm.

The insertion position indicator unit 22 is configured to indicate that the position of the inserted tampon is appropriate when the strand tail end 12d falls within 70 mm to 90 mm from the end of the position confirmation sheet at the body side. In the present embodiment a dimension Lc in a longitudinal direction of the absorber 11 is 45 mm and a dimension in a longitudinal direction L of the inner tube 3 is 70 mm. In the applicator-type tampon 1, a length of the strand member 12 disposed in the vagina when the absorber 11 is disposed at an appropriate position is about 43 mm. A dimension Ld in the longitudinal direction L of the strand member 12 is 113 mm to 133 mm. Therefore, the length of the strand member that extends from the vaginal opening desirably is 70 mm to 90 mm.

Preferably, the absorber 11 is inserted in such a manner that a distance from the vaginal opening to the tip 11a of the absorber 11 is at least 70 mm, ranging preferably from 70 mm to 150 mm. When the tip 11a of the absorber 11 falls within this range, a user rarely feels discomfort.

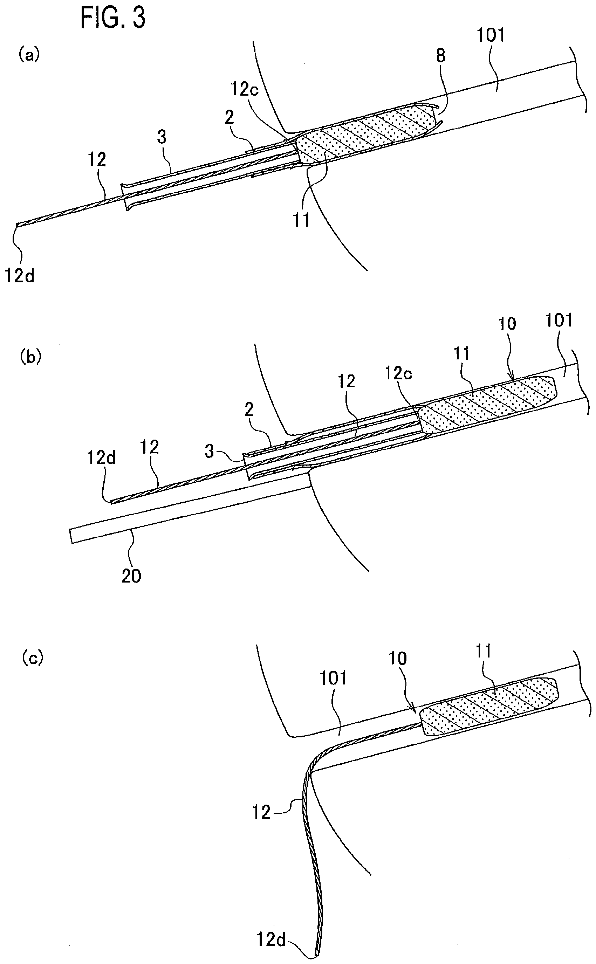

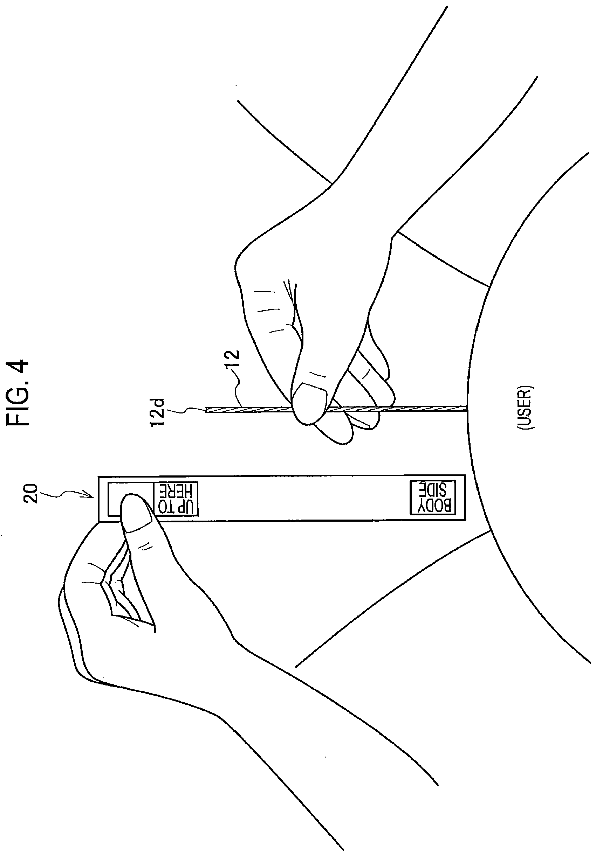

Next, with reference to Fig. 3 and Fig. 4, use modes of the position confirmation sheet configured as above will be explained. Fig. 3 is a diagram showing an applicator-type tampon and the position confirmation sheet in use. At the time of using the absorber 11, a user inserts the outer tube 2 into the appropriate position of the vagina 101. Fig. 3(a) shows a state where the outer tube 2 is inserted into the appropriate position of the vagina 101.

Next, after inserting the outer tube 2 into a predetermined position in the vagina, the user presses up the inner tube 3 toward the outer tube 2. The absorber 11 is extruded from the push-out aperture 8 of the outer tube 2, and the absorber 11 is disposed inside the vagina 101. Fig. 3(b) is a diagram showing a state where the absorber 11 is extruded into the vagina.

The user disposes the position confirmation sheet 20 according to the disposition indicator unit 21, and checks the position of the strand tail end 12d of the strand member 12 relative to the insertion position indicator unit 22. Fig. 4 schematically shows a state from a user's view point where the position confirmation sheet 20 is aligned according to the disposition indicator unit 21. In the state shown in Fig. 4, when the strand tail end 12d falls on the appropriate position indicator unit 22a, a user can assess that the absorber 11 is disposed at an appropriate position, and when the strand tail end falls on the inappropriate position indicator unit 22b, the user assesses that the absorber is disposed at an inappropriate position.

Then, the user extracts the outer tube 2 and the inner tube 3 from the vagina. The absorber 11 is disposed in the vagina, and the strand member at the strand tail end side is disposed outside the body. Fig. 3(c) shows a state where the absorber is disposed in the vagina.

By using the position confirmation sheet 20, the user can check whether or not the absorber 11 is disposed appropriately in the body based on the position of the strand tail end 12d disposed outside the body relative to the insertion position indicator unit 22.

By confirming that the absorber 11 is disposed at an appropriate position, a user can free from anxiety, for instance. On the other hand, by finding that the absorber is disposed at an inappropriate position, the user would correct the position of the sanitary tampon, where necessary. Further, this finding may help the user insert a sanitary tampon appropriately from the next time on. In this way, by disposing the absorber 11 at an appropriate position, the user may have less discomfort, using a sanitary tampon comfortably. In particular, for a beginner not accustomed to the handling of the sanitary tampon, checking the position of the inserted tampon can relieve anxiety over the position of the inserted tampon.

Further, unlike the conventional sanitary tampon with such a configuration that the strand member 12 inserted into the body is colored, there is no foreign object such as a colored portion or a marker to be inserted, so that user's sanitary concern can be eliminated.

Further, a user does not have to hold the center of the strand member or peek at an area near the vaginal opening because it is possible to check the position of the absorber by aligning the strand tail end 12d with the position confirmation sheet, which is the farthest part of the sanitary tampon. Thus, in checking the position of the absorber, a user's hand does not get dirty, and uncomfortable action such as peeking at the vaginal opening is not necessary, whereby a user's psychological burden can be alleviated.

It is noted that a method of using the position confirmation sheet and a method of checking the position of the absorber are preferably indicated on a container that contains the sanitary tampon or an accessory of the sanitary tampon such as an explanatory note of the sanitary tampon.

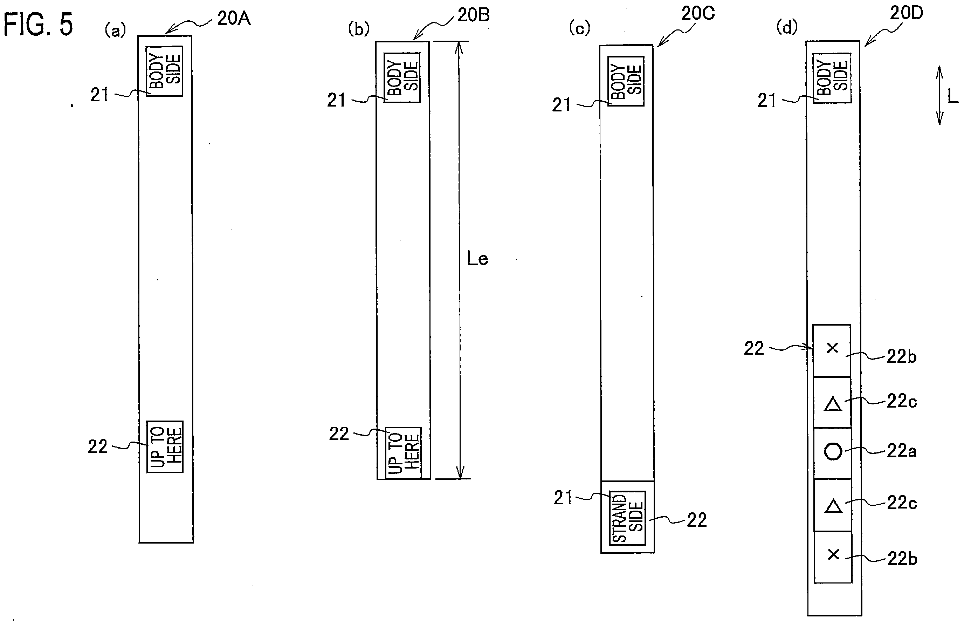

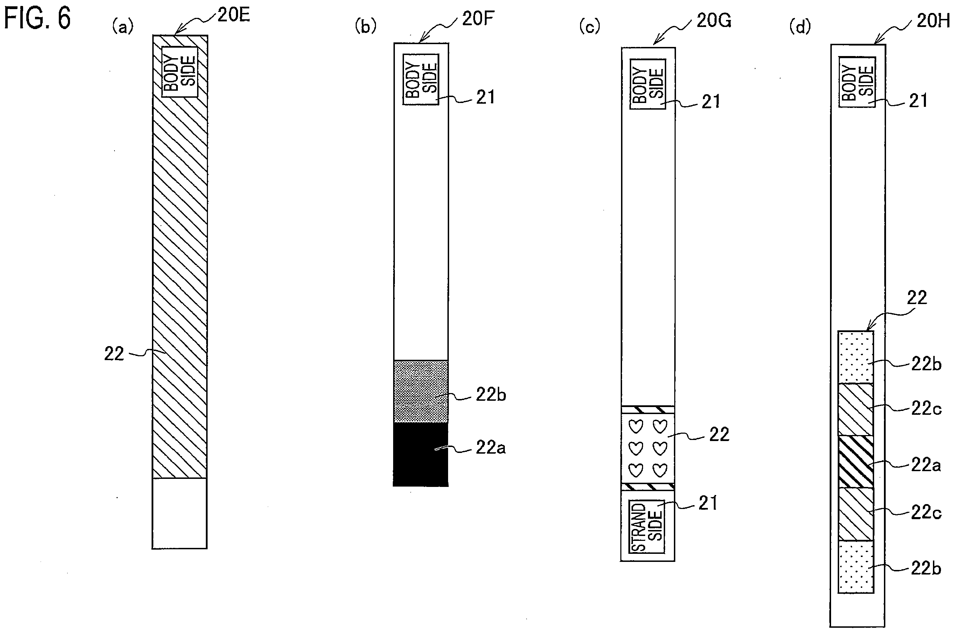

Next, with reference to Fig. 5 and Fig. 6, a position confirmation sheet according to a first modification to an eighth modification will be explained. It is noted that in the explanation of the modifications, only configurations different from that of the first embodiment will be explained, and similar configurations will be simply referred to by using identical reference numbers. Fig. 5(a) to Fig. 5(d) are plan views of the position confirmation sheet according to the first modification to the fourth modification, and Fig. 6(a) to Fig. 6(d) are plan views of the position confirmation sheet according to the fifth modification to the eighth modification.

A position confirmation sheet 20A according to the first modification is configured such that, as shown in Fig. 5(a), the insertion position indicator unit 22 is configured only by the appropriate position indicator unit 22a without the inappropriate position indicator unit.

A position confirmation sheet 20B according to the second modification is configured such that, as shown in Fig. 5(b), the insertion position indicator unit 22 is located at a rear end of the position confirmation sheet in the insertion direction and is configured to indicate the appropriate position of the end of the strand member disposed outside the body by the length in the insertion direction of the position confirmation sheet. A dimension Le in a longitudinal direction of the position confirmation sheet according to the second modification is 70 mm to 90 mm.

With the position confirmation sheet according to the second modification, it is possible to check whether or not the absorber is appropriately inserted by observing whether or not the strand tail end falls at the end of the position confirmation sheet. Therefore, even when it is not possible to visually check the position of the strand tail end, it is possible to check by the feel of touch whether or not the strand tail end falls at the end of the position confirmation sheet, allowing a use in a place such as a relatively dark toilette.

In a position confirmation sheet 20C according to the third modification, as shown in Fig. 5(c), a plurality of disposition indicator units 21 are provided, and the insertion position indicator unit 22 is located at a predetermined region partitioned by a line.

In a position confirmation sheet 20D according to the fourth modification, as shown in Fig. 5(d), a plurality of insertion position indicator units 22 are disposed adjacently in a longitudinal direction L, which is an insertion direction of the absorber. The plurality of insertion position indicator units is configured in such a manner that each unit indicates a degree of properness of the insertion of the absorber in the body.

The insertion position indicator unit 22 includes: an appropriate position indicator unit 22a; an inappropriate position indicator unit 22b; and a middle position indicator unit 22c disposed between the appropriate position indicator unit 22a and the inappropriate position indicator unit 22b. Thus, by indicating a degree of properness of the insertion stepwise by the plurality of insertion position indicator units, a user can check an inserted state of the absorber where the insertion depth is not enough and how far the absorber should be inserted to reach an appropriate position, for instance.

In a position confirmation sheet 20E according to the fifth modification, as shown in Fig. 6(a), the insertion position indicator unit 22 is patterned. The patterning allows the user to instinctively comprehend whether or not the absorber is inserted at a correct position. It is noted that the pattern may be provided to the insertion position indicator unit 22 and may also be provided to a region other than the insertion position indicator unit.

In a position confirmation sheet 20F according to the sixth modification, as shown in Fig. 6(b), the insertion position indicator unit 22 is colored. The position confirmation sheet 20F may be colored with at least one color so as to be visually distinguishable. To be visually distinguishable means that there is a difference in an aspect such as color, pattern, texture, reflectance, opacity, semitransparency, milky-whiteness, fluorescence, luminescence, phosphorescence, chemoluminescence, brightness or the like.

Further, the insertion position indicator unit of the position confirmation sheet may be colored with two or more colors, and the appropriate position indicator unit and the inappropriate position indicator unit may be indicated differently by different color. Examples of a method of coloring the position confirmation sheet may include photogravure printing, ink jet printing, lamination, and other methods.

When the insertion position indicator unit 22 is colored or patterned, it is possible to confirm the insertion position of the absorber 11 only by comprehending the color or the pattern, and thus, even a user not capable of reading a text can favorably use the sheet. Further, with the colored or patterned insertion position indicator unit 22, it is possible to simplify an explanatory note on a method of using the position confirmation sheet, for instance.

In a position confirmation sheet 20G according to the seventh modification, as shown in Fig. 6(c), the insertion position indicator unit 22 is patterned with a figure of a heart. The pattern may be of various figures such as stripe, heart pattern, dot pattern and a star pattern.

A position confirmation sheet 20H according to the eighth modification is configured such that a friction coefficient of the insertion position indicator unit 22 is different from a friction coefficient of a region other than the insertion position indicator unit. From a difference in the feel of touch, the user can check haptically whether or not the position of the absorber is appropriate.

In the insertion position indicator unit 22 of the position confirmation sheet 20H, at least one rugged unit is formed. It is noted that the rugged unit may be formed only in the appropriate position indicator unit of the insertion position indicator unit, or formed only in the inappropriate position indicator unit.

The rugged unit may be formed through embossing, for example, or by applying rubber, urethane, or hot-melt adhesive, or by other methods.

It is noted that the above-described position confirmation sheets may be provided to each sanitary tampon to be contained in a sanitary tampon container, or the position confirmation sheet may be contained in the container as a sheet that includes a plurality of continuous position confirmation sheets configured to be separated via a perforation line.

### (Second embodiment)

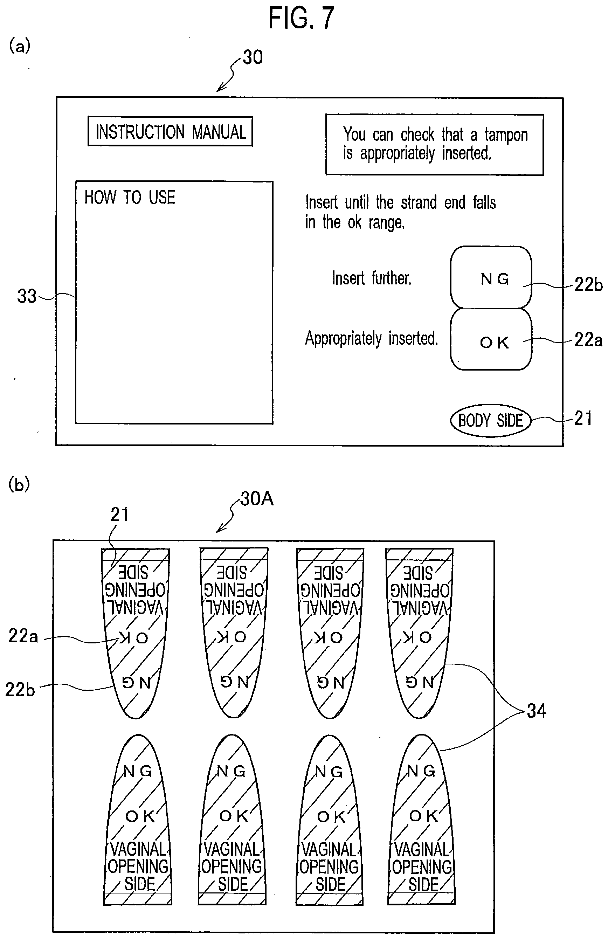

Next, with reference to Fig. 7, an explanatory note 30 of a sanitary tampon according to a second embodiment will be explained. The explanatory note of the sanitary tampon according to the second embodiment is contained in a container that contains sanitary tampons and shows to a user usage instructions of a sanitary tampon. The explanatory note is made of paper comprising a position confirmation sheet, and includes a usage indication unit 33 for explaining a method of using a sanitary tampon.

Fig. 7(a) is an embodiment in which the disposition indicator unit 21 and the insertion position indicator unit 22 are arranged on a face on which a usage method is indicated. On the explanatory note 30, the usage indication unit 33, the disposition indicator unit 21, and the appropriate position indicator unit 22a and the inappropriate position indicator unit 22b as the insertion position indicator unit are printed.

By providing the insertion position indicator unit 22 in the explanatory note 30, a user can check whether or not the absorber 11 is inserted at an appropriate position while reading the usage method of a sanitary tampon. In particular, it enables a beginner, who is not accustomed to handling sanitary tampon, to use a sanitary tampon by referring to the usage method, and at the same time to check the insertion position.

Fig. 7(b) is an embodiment in which the disposition indicator unit 21 and the insertion position indicator unit 22 are arranged on the reverse side of the face on which a usage method is indicated. The explanatory note of the sanitary tampon shown in Fig. 7(b) is an explanatory note for a sanitary tampon not having an applicator. To the explanatory note, a finger sack 34 is attached, which is to be worn on a user's finger in inserting the absorber 11 into the body.

The finger sack 34 is made from a polyethylene sheet, and is bonded detachably to the explanatory note. On the top surface of the finger sack, the disposition indicator unit 21, and the appropriate position indicator unit 22a and the inappropriate position indicator unit 22b are printed. The finger sack functions as the position confirmation sheet.

The explanatory note may be produced by the following method. Firstly, an indication unit such as the disposition indicator unit 21 and the insertion position indicator unit 22 are printed on a polyethylene sheet. Next, the polyethylene sheet is heated on a sheet of the explanatory note so that the explanatory note and the polyethylene sheet are bonded. Thereafter, a mold having a shape of a finger sack is used to fuse the polyethylene sheet according to the shape. By fusing the polyethylene sheet in this way, the polyethylene sheet is cut in the shape of finger sack to form a finger sack and to bond the finger sack to the explanatory note.

### (Third embodiment)

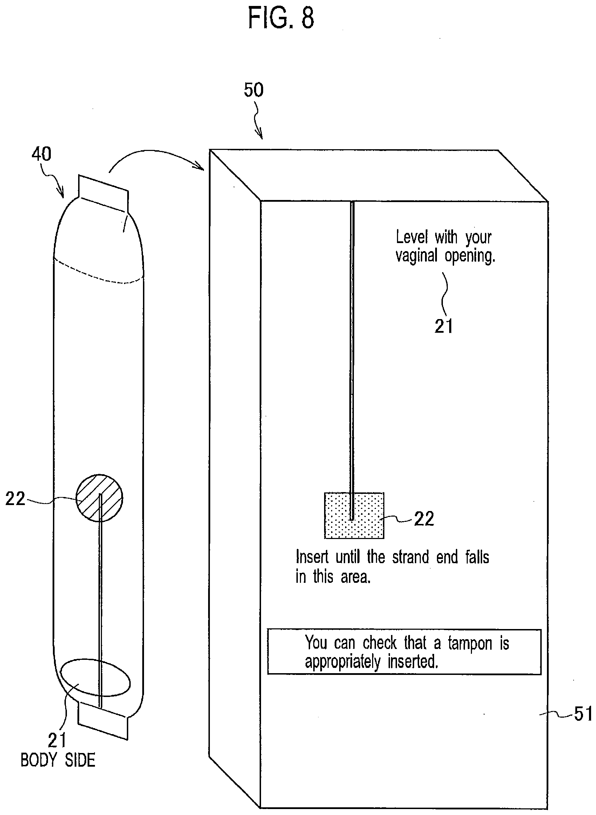

Next, with reference to Fig. 8, a package bag 40 of a sanitary tampon according to a third embodiment and a container 50 of sanitary tampons will be explained. The package bag 40 of the sanitary tampon comprises a position confirmation sheet, and a sanitary tampon individually. In the package bag, an unillustrated sanitary tampon is contained. On the outer surface of the package bag, the disposition indicator unit 21 and the insertion position indicator unit 22 are printed.

The container 50 of the sanitary tampon contains a plurality of sanitary tampons, and on its outer surface, the position confirmation sheet is provided. The position confirmation sheet is arranged so as to cover the outer surface of the container. On a position confirmation sheet 51, the disposition indicator unit 21 and the insertion position indicator unit 22 are printed. It is noted that in the present embodiment, the position confirmation sheet is arranged so as to cover the outer surface of a box-like object that contains the sanitary tampons, but the disposition indicator unit 21 and the insertion position indicator unit 22 may be printed on the box-like object itself that contains the sanitary tampons.

As in the second embodiment and the third embodiment, a user can check whether or not the absorber is disposed appropriately by using an accessory such as the package bag, the container, and the explanatory note of the sanitary tampon provided with the disposition indicator unit and the insertion position indicator unit of the position confirmation sheet

With the disposition indicator unit and the insertion position indicator unit of the position confirmation sheet being provided to an accessory such as the package bag, the container, and the explanatory note of the sanitary tampon, the number of components is smaller than that in the configuration where the position confirmation sheet is separately provided from the accessory, whereby work efficiency is improved and costs are reduced.

It is noted that the disposition indicator unit and the insertion position indicator unit may be integrated with an accessory, or configured to be separable via a perforation.

As described above, although the content of the present invention was disclosed through the embodiments of the present invention, the descriptions and drawings that form a part of this disclosure are not to be considered as limitation to the present invention. From this disclosure, a variety of alternate embodiments, examples, and applicable techniques will become apparent to one skilled in the art.

### [Industrial Applicability]

A position confirmation sheet, an explanatory note of a sanitary tampon, a package bag of a sanitary tampon, and a container of a sanitary tampon are provided. By disposing the position confirmation sheet according to a disposition indicator unit and checking a position of the end of the strand member relative to an insertion position indicator unit, a user can confirm that an absorber is disposed appropriately in a body.

### [Reference Signs List]

1 applicator-type tampon,
2 outer tube,
3 inner tube,
8 push-out aperture,
10 sanitary tampon,
11 absorber,
11a tip,
11b tail end,
12 strand member,
12c strand tip,
12d strand tail end,
20, 20A, 20B, 20C, 20D, 20E, 20F, 20G, 20H position confirmation sheet,
21 disposition indicator unit,
22 insertion position indicator unit,
22a appropriate position indicator unit,
22b inappropriate position indicator unit,
30 explanatory note of sanitary tampon,
33 usage indication unit,
34 finger sack,
40 package bag of sanitary tampon,
50 container of sanitary tampon,
51 position confirmation sheet

## Claims

1. A position confirmation sheet for a sanitary tampon (10), wherein the sanitary tampon comprises an absorber (11) and a strand member (12) extending from a tail end of the absorber in a direction of insertion thereof into a vagina, and the position confirmation sheet is to be used for checking a position of an end of the strand member (12) disposed outside a body with the absorber (11) being inserted into the body, comprising:
a disposition indicator unit (21) that indicates a position for aligning the confirmation sheet along the strand member (12) disposed outside the body with the absorber (11) being inserted into the body; and
an insertion position indicator unit (22) that indicates an appropriate position of the end of the strand member (12) disposed outside the body when the absorber (11) is appropriately inserted into the body and the position confirmation sheet is aligned along the strand member (12) according to the disposition indicator unit (21).

2. The position confirmation sheet of a sanitary tampon according to claim 1, wherein
a plurality of the insertion position indicator units (22) are disposed adjacently in the insertion direction of the absorber, and the plurality of insertion position indicator units each indicates a degree of properness of the insertion of the absorber (11) into the body.

3. The position confirmation sheet of a sanitary tampon according to claim 1 or 2, wherein
the insertion position indicator unit (22) has a color or a pattern different from that in a region other than the insertion position indicator unit (22) of the position confirmation sheet.

4. The position confirmation sheet of a sanitary tampon according to claim 1 or 2, wherein a friction coefficient of the insertion position indicator unit (22) is different from a friction coefficient of a region other than the insertion position indicator unit (22) of the position confirmation sheet.

5. The position confirmation sheet of a sanitary tampon according to claim 1, wherein the insertion position indicator unit (22) is provided at a rear end of the position confirmation sheet in the insertion direction and the position confirmation sheet is configured to indicate an appropriate position of the end of the strand member (12) disposed outside the body by a length thereof in the insertion direction.

6. A package bag that individually contains a sanitary tampon, the package bag (40) comprising the position confirmation sheet according to any one of claims 1 to 5, wherein the insertion position indicator unit (22) is disposed at an outer surface of the package bag (40).

7. A container that contains a plurality of sanitary tampons, the container (50) comprising the position confirmation sheet according to any one of claims 1 to 5, wherein the insertion position indicator unit (22) is disposed at an outer surface of the container (50).

## Patentansprüche

1. Positionsbestätigungslage für ein Hygienetampon (10), wobei das Hygienetampon einen Absorber (11) und ein Strangelement (12), das sich von einem Endstück des Absorbers in eine Richtung seines Einführens in eine Vagina erstreckt, umfasst und die Positionsbestätigungslage zum überprüfen einer Position eines Endes des Strangelements (12) ist, das außerhalb eines Körpers, wenn der Absorber (11) in den Körper eingeführt ist, angeordnet ist, umfassend:
eine Anordnungsanzeigeeinheit (21), die eine Position zum Ausrichten der Positionsbestätigungslage entlang des außerhalb des Körpers angeordneten Strangelements (12), wenn der Absorber (11) in den Körper eingeführt ist, anzeigt; und
eine Einführpositionsanzeigeeinheit (22), die eine geeignete Position des Endes des außerhalb des Körpers angeordneten Strangelements (12) anzeigt, wenn der Absorber (11) geeignet in den Körper eingeführt ist, und die Positionsbestätigungslage entlang des Strangelements (12) gemäß der Anordnungsanzeigeeinheit (21) ausgerichtet ist.

2. Positionsbestätigungslage eines Hygienetampons nach Anspruch 1, wobei
eine Vielzahl der Einführpositionsanzeigeeinheiten (22) benachbart in der Einführrichtung des Absorbers angeordnet ist und die Vielzahl von Einführpositionsanzeigeeinheiten jeweils einen Grad der Korrektheit der Einführung des Absorbers (11) in den Körper anzeigt.

3. Positionsbestätigungslage eines Hygienetampons nach Anspruch 1 oder 2, wobei
die Einführpositionsanzeigeeinheit (22) eine Farbe oder ein Muster hat, das verschieden zu dem in einem anderen Bereich als die Einführpositionsanzeigeeinheit (22) der Positionsbestätigungslage ist.

4. Positionsbestätigungslage eines Hygienetampons nach Anspruch 1 oder 2, wobei ein Reibungskoeffizient der Einführpositionsanzeigeeinheit (22) verschieden von einem Reibungskoeffizienten eines anderen Bereichs als die Einführpositionsanzeigeeinheit (22) der Positionsbestätigungslage ist.

5. Positionsbestätigungslage eines Hygienetampons nach Anspruch 1, wobei die Einführpositionsanzeigeeinheit (22) in der Einführrichtung an einem hinteren Ende der Positionsbestätigungslage vorgesehen ist und die Positionsbestätigungslage ausgebildet ist, eine geeignete Position des Endes des Strangelements (12), das mit einer Länge davon in der Einführrichtung außerhalb des Körpers angeordnet ist, anzuzeigen.

6. Verpackungsbeutel, der einzeln einen Hygienetampon enthält, wobei der Verpackungsbeutel (40) die Positionsbestätigungslage nach einem der Ansprüche 1 bis 5 umfasst, wobei die Einführpositionsanzeigeeinheit (22) an einer äußeren Oberfläche des Verpackungsbeutels (40) angeordnet ist.

7. Behälter, der eine Vielzahl von Hygienetampons enthält, wobei der Behälter (50) die Positionsbestätigungslage nach einem der Ansprüche 1 bis 5 umfasst, wobei die Einführpositionsanzeigeeinheit (22) an einer äußeren Oberfläche des Behälters (50) angeordnet ist.

## Revendications

1. Couche de confirmation de position d'un tampon menstruel (10), dans laquelle le tampon menstruel comprend un absorbeur (11) et un élément formant cordon (12) s'étendant d'une extrémité arrière de l'absorbeur dans une direction d'insertion de celui-ci à l'intérieur du vagin et la couche de confirmation de position doit être utilisée pour vérifier la position d'une extrémité de l'élément formant cordon (12) disposé à l'extérieur du corps avec l'absorbeur (11) étant inséré à l'intérieur du corps, comprenant :
une unité indiquant la disposition (21) qui indique une position pour aligner la couche de confirmation le long de l'élément formant cordon (12) disposé à l'extérieur du corps avec l'absorbeur (11) étant inséré à l'intérieur du corps ; et
une unité indiquant la position d'insertion (22) qui indique une position appropriée de l'extrémité de l'élément formant cordon (12) disposé à l'extérieur du corps lorsque l'absorbeur (11) est inséré de manière appropriée à l'intérieur du corps et la couche de confirmation de position est alignée le long de l'élément formant cordon (12) selon l'unité indiquant la disposition (21).

2. Couche de confirmation de position d'un tampon menstruel selon la revendication 1, dans laquelle
une pluralité d'unités indiquant la position d'insertion (22) est disposée de manière adjacente dans la direction d'insertion de l'absorbeur et la pluralité d'unités indiquant la position d'insertion indique chacune un degré d'exactitude de l'insertion de l'absorbeur (11) à l'intérieur du corps.

3. Couche de confirmation de position d'un tampon menstruel selon la revendication 1 ou 2, dans laquelle
l'unité indiquant la position d'insertion (22) a une couleur ou un motif différent(e) de celle/celui situé(e) dans une zone autre que l'unité indiquant la position d'insertion (22) de la couche de confirmation de position.

4. Couche de confirmation de position d'un tampon menstruel selon la revendication 1 ou 2, dans laquelle le coefficient de frottement de l'unité indiquant la position d'insertion (22) est différent d'un coefficient de frottement d'une zone autre que l'unité indiquant la position d'insertion (22) de la couche de confirmation de position.

5. Couche de confirmation de position d'un tampon menstruel selon la revendication 1, dans laquelle l'unité indiquant la position d'insertion (22) est prévue à une extrémité arrière de la couche de confirmation de position dans la direction d'insertion et la couche de confirmation de position est configurée pour indiquer une position appropriée de l'extrémité de l'élément formant cordon (12) disposé à l'extérieur du corps par une longueur de celui-ci dans la direction d'insertion.

6. Sachet d'emballage qui contient individuellement un tampon menstruel, le sachet d'emballage (40) comprenant la couche de confirmation de position selon l'une quelconque des revendications 1 à 5, dans lequel l'unité indiquant la position d'insertion (22) est disposée sur une surface externe du sachet d'emballage (40).

7. Boîte contenant une pluralité de tampons menstruels, la boîte (50) comprenant la couche de confirmation de position selon l'une quelconque des revendications 1 à 5, dans laquelle l'unité indiquant la position d'insertion (22) est disposée sur une surface externe de la boite (50).
